# EUROPEAN PATENT APPLICATION

(11) **EP 0 597 553 A1**
(43) Date of publication of application: **18.05.1994**
(21) Application number: 93203168.5
(22) Date of filing: 12.11.1993
(51) Int. Cl.: A61F 2/32, A61L 27/00, A61F 2/36

(54) **Method of attaching a modular femoral head to the neck of a hip stem prosthesis for the preventation of corrosion therebetween**

(30) Priority: 12.11.1992 US 975294
(71) Applicant: Bristol-Myers Squibb Company, New York, N.Y. 10154 (US)
(72) Inventor: Devanathan, Thirumalai, Warsaw, IN 46580 (US)
(74) Representative: Mays, Julie

(57) **Abstract**

The method includes the steps of providing a thin layer of a thermoplastic polymer on the outer mating surface of the femoral neck 8. To assemble the femoral head 12 and neck 8, the head 12 is placed onto the neck 8 and the assembly is heated to a temperature of approximately 800 degrees Fahrenheit. At this temperature, the thermoplastic polymer becomes molten and exhibits exceptional adhesive characteristics. Once the thermoplastic is in its molten state, the femoral head 12 is impacted onto the neck by known instrumentation. Impacting provides a tight mechanical connection between the mating parts. Once impacted, the assembly is permitted to cool to ambient temperature which transforms the thermoplastic polymer into its solid state. The thermoplastic polymer chemically bonds the mating parts together and exhibits a strength at the junction around 10 ksi.

The use of the thin layer of thermoplastic polymer also forms a uniform contact surface between the mating parts to allow for stress transfer across the entire mating surface and thereby reduces the overall transfer of stress per unit area. The bond created between the mating parts further forms a barrier to moisture to prevent corrosion or fretting between the mating surfaces.

## Description

### Field of the Invention

This invention relates to modular orthopaedic implants and more particularly to a method of attaching a modular femoral head to the neck of a hip stem implant.

### Summary of the Invention

Modular prosthetic implants have become common in the field of orthopaedics to offer a multiplicity of combinations to the surgeon in order to obtain the proper fit for the patient. It is also well known in the orthopaedic field to use varying types of materials for prostheses which are suitable for implantation in the body. It has been known to use the following combinations of materials for modular joint combinations, such as a titanium alloy component mated with a titanium alloy component or a cobalt alloy component with a cobalt alloy component or a titanium alloy component with a cobalt alloy component. It is noted that suitable combinations of materials are not limited to those noted. It has been recognized that some surface changes, such as various types of corrosion (such as pitting, crevice corrosion, mechanical fretting, and galvanic corrosion) and wear can occur between modular connections. While this phenomenon has been observed in modular components made of both similar and dissimilar metals, and although such observed corrosion and wear may not be of clinical or mechanical significance, nevertheless, there seems to be concern about this corrosion and wear and a desire to reduce or minimize its occurrence, especially between modular connections of dissimilar metals. This corrosion and wear may be partly due to slight motion (micro motion) between the two mating, mechanically secured, metal surfaces that results from functional weight-bearing of the implanted modular prosthesis.

The following devices are known in the art: U.S. Patent 3,943,576 to Sivash discloses a prosthesis with a first portion made from a cobalt alloy and a second portion made from titanium or a titanium alloy, and wherein the first and second portions are in physical contact. The cobalt alloy hip ball 28 is mechanically secured to the titanium neck 26 of the hip stem by shrinking the hip ball on the neck. The cobalt alloy inserts 30 are mechanically secured to the titanium acetabulum prosthesis 10 by hammering down or riveting projections 32.

EP 0 193 681 A2 to Elloy and U.S. Patents 4,908,034 to Weightman et al., 4,404,691 to Buning et al., and 4,227,265 to Frey each disclose modular prosthetic implants having a plastic interpositional component between two other components. Elloy states that it provides the interposing plastic component to keep separate those components of a prosthesis being made of incompatible materials.

U.S. Patent 5,057,111 to Park discloses a bone fixation plate having a polymer member fitted in a hole in the plate between the plate and a bone screw.

U.S. Patents 5,015,257 to Crowninshield et al., 4,921,500 to Averill et al., and 4,846,841 to Oh each disclose metal interpositional members mechanically secured via a friction fit between two other components.

U.S. Patent 4,032,994 to Frey discloses a metal sleeve 3 which is mechanically secured to joint head 1 via a self-locking taper. The sleeve 3 is rotatably and axially movably mounted via bore 4 on pin 5.

U.S. Patent 4,995,883 to Demane et al. discloses a modular hip prosthesis with a wide variety of modular components which are mechanically secured in a variety of ways, either via taper locking (such as at the conical junctions between the head 27, the sleeves or extension members 45 or 50, and the neck 14) or via a mechanical, threaded interconnection or a mechanical pinning interlock.

German Patent DT 26 18 763 to Ribonet discloses a modular prosthesis having a ceramic, aluminum oxide ball which is coupled to a titanium shaft via a blind hole in the ball into which a titanium insert can be soldered with the end 6 of the shaft fastened to it so that any rotational or relative translational motion is impossible. Ribonet also discloses a means of locking the insert to the femoral neck via a crosspin, threads, or keyway and pin.

U.S. Patent 4,687,488 to Frey discloses a joint head 5 which is secured to the base 4 of the sleeve 2 by means of a weld 12. The weld 12 is at the outer junction of the sleeve and head. The cover 8 of the sleeve is also secured to the sleeve 2 by a circumferential weld 11. The outer walls of the sleeve do not communicate with the interior of the shell and are not supported in the interior of the shell along the length of the sleeve wall.

U.S. 4,969,907 to Koch et al. discloses an implant with a cobalt alloy body having a metal coating applied thereto with a metal structural element secured to the coating. The coating and structural element may be titanium, tantalum, niobium, or alloys thereof.

UK Patent GB 2 142 544B to Medcraft discloses an orthopaedic implant which utilizes diffusion bonding of a mesh-form sheet material to a surface of a substrate.

UK Patent Application GB 2 223 174A to Jacobs et al. discloses base portion 10 with an additional portion 20 secured thereto. The additional portion 20 includes at least one post extending therefrom and adapted to be received and mechanically secured in a corresponding reverse tapered hole in the base portion. The additional portion 20 may include a substrate 22 with a porous surface 24 bonded or secured thereto. The base portion 10 may be a cobalt material while the substrate 22 and porous surface 24 may be titanium material. While the materials in Jacobs et al. are not restricted to this particular combination, it is noted that it is the similar materials in Jacobs et al. that are bonded together and the dissimilar materials that are mechanically bonded together.

### Summary of the Invention

The method of this invention eliminates the above corrosion concerns and provides for a stable bond between the femoral head and neck to prevent fretting or micro motion therebetween. The method further provides a moisture barrier to further limit corrosion between the bonded parts. The method of this invention has application to bonding either a femoral head to the neck of a hip stem implant or to bonding an interpositional coupler between the femoral head and neck of the hip stem.

The method includes the steps of providing a thin layer of a thermoplastic polymer on the outer mating surface of the femoral neck. The femoral head and neck are assembled and heated to a temperature of approximately 800 degrees Fahrenheit. At this temperature, the thermoplastic polymer becomes molten and exhibits exceptional adhesive characteristics. Once the thermoplastic is in its molten state, the femoral head is impacted onto the neck by known instrumentation. Impacting provides a tight mechanical connection between the mating parts. Once impacted, the assembly is permitted to cool to ambient temperature which transforms the thermoplastic polymer into its solid state. The thermoplastic polymer bonds the mating parts together and exhibits a bond strength at the junction around 10 ksi.

In the preferred embodiment, the thermoplastic polymer of choice is from the polyaryletherketone family (PAEK). Specifically, a PAEK sold by BASF under the trade name Ultrapek@ A3000. The PAEK polymers have shown through experimentation to exhibit strong adhesive bonds with molybdenum alloy (Co-Cr-Mo) and titanium-vanadium-aluminum alloy (Ti-6A1-4V) commonly used for implant materials.

The use of the thin layer of thermoplastic polymer forms a uniform contact surface between the mating parts to allow for stress transfer across the entire mating surface and thereby reduces the overall contact stress per unit area. The bond created between the mating parts further forms a barrier to moisture to prevent corrosion or fretting between the mating surfaces. This also reduces the need to obtain extremely tight tolerances in taper/ball construction.

The method of the invention has application to the bonding of dissimilar metals at the head to neck junction or in the bonding of similar metals at the head to neck junction with the same results. In this manner, the designer is free to design an implant and take advantage of the qualities of cobalt chrome for the femoral head and titanium for the hip stem without concern of unwanted corrosion or fretting between the parts.

Accordingly, it is an object of this invention to provide for a novel method of bonding the femoral head to the neck of a hip stem implant using a thermoplastic polymer.

Another object of the invention is to provide for a method of bonding the femoral head to the neck of a hip stem implant using a thermoplastic polymer which prevents moisture from entering the mating junction between the parts since semi crystalline polymers like PEAK's are very moisture resistant.

Another object of the invention is to provide for a method of bonding the femoral head to the neck of a hip stem implant using a thermoplastic polymer which provides an adhesive bond between the mating parts.

Another object of the invention is to provide for a method of bonding the femoral head to the neck of a hip stem implant using a thermoplastic polymer which provides for the even transfer of stress across the bond to thereby reduce the overall stress per unit area at the bond.

Still another object of the invention is to provide for a method of bonding the femoral head to the neck of a hip stem implant using a thermoplastic polymer having a bond strength of around 10 ksi.

Other objects of the invention will become apparent upon a reading of the following description taken with the accompanying drawings.

### Brief Description of the Drawings

Fig. 1 is a cross sectional view illustrating a portion of a hip stem having a neck which terminates in a truncated cone shape. A femoral head is illustrated as including a cavity shaped to closely match the neck of the hip stem. Fig. 1 represents the prior art.
Fig. 2 illustrates the initial step of the method wherein a thermoplastic polymer fiber is wound around the mating surface of the implant neck. The thermoplastic fiber is wound in a single layer.
Fig. 3 illustrates the next step in the method wherein the implant is heated to cause the polymer to melt forming a smooth layer of polymer on the mating surface of the neck.
Fig. 4 illustrates the next step of the method of the invention wherein a femoral head is inserted onto the neck of the hip stem. The mating surface of the neck carries the thin layer of thermoplastic polymer. The assembly is heated to a temperature around 800 degrees Fahrenheit.
Fig. 5 illustrates the next step of the method of the invention wherein after the assembly is heated and the thermoplastic polymer is in a molten state, the femoral head is impacted upon the neck in a known manner.
Fig. 6 illustrates the use of the method of the invention associated with a femoral head, the neck of a hip stem and interpositional coupler.

### DESCRIPTION OF THE PREFERRED EMBODIMENTS

The preferred embodiment herein described is not intended to be exhaustive or to limit the invention to the precise form disclosed. Rather, it is chosen and described to best explain the invention so that others skilled in the art might utilize their teachings.

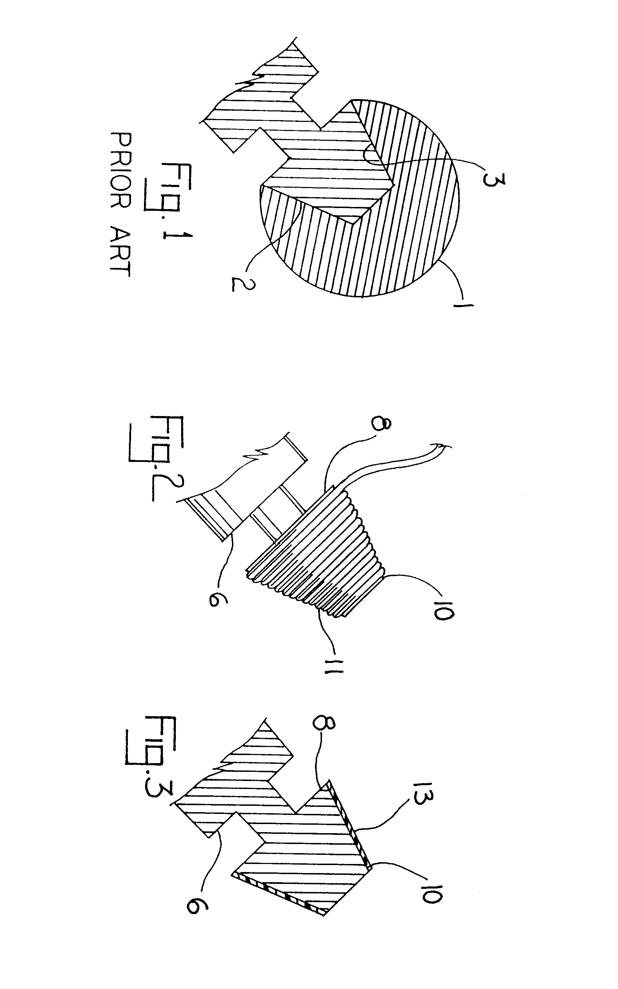

Figure 1 is provided merely to illustrate the prior art mechanical connection between the femoral head 1 and the neck 2. The neck 2 of the hip stem implant (partially shown) is shaped in a generally truncated cone configuration as illustrated. Head 1 includes a cavity 3 shaped to closely match the femoral neck. It is preferred in the art to design the neck and femoral head cavity so as to be closely matched to one another to limit movement along the mating surfaces. The femoral head is impacted onto the neck using known impaction techniques. The head and neck may be of the same material or of different materials such as a cobalt chrome head and a titanium neck. It is such a combination of dissimilar metals that lends itself especially to galvanic corrosion which may be enhanced by micro motion along the mating surfaces with the inclusion of moisture. To assist in minimizing the effects of corrosion, manufacturers may provide an interpositional coupler formed from the same material as the neck. The coupler may be metallurgically bonded to the head in an attempt to prevent relative motion therebetween. The coupler is typically formed from the same material as the neck with the idea that similar metals in contact experience less corrosion problems.

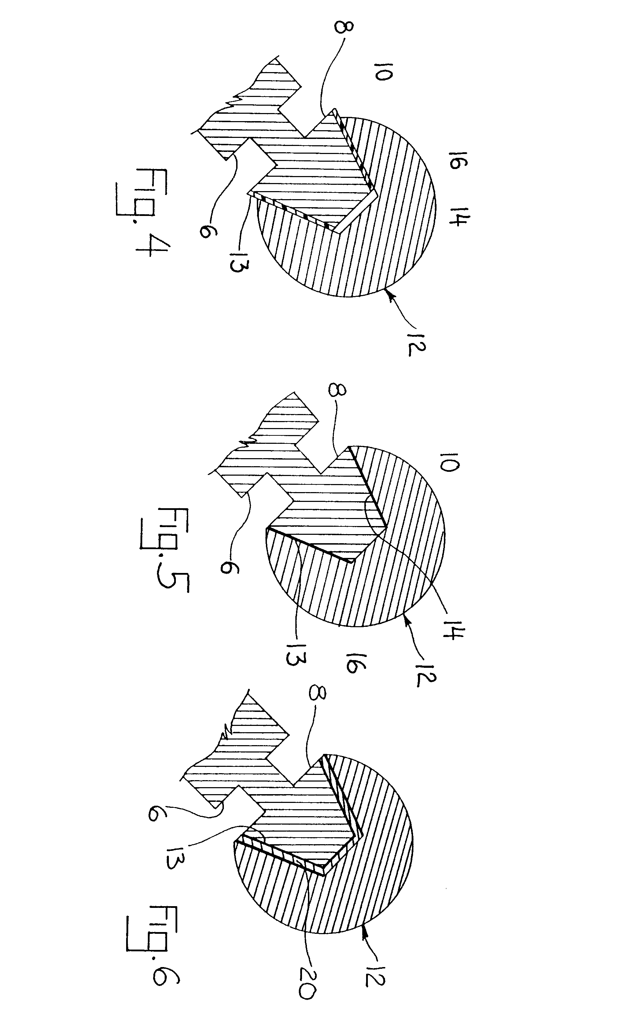

The method of this invention and its use for bonding the femoral head and neck is illustrated in figures 2 through 6. A portion of a hip stem implant 6 is illustrated and includes a neck 8 having a tapered mating surface 10. A femoral head 12 is also illustrated and includes a tapered cavity 14 having a mating surface 16 (see Figs. 4 and 5). Initially a thin film of a thermoplastic polymer is placed on the mating surface 10 of neck 8. In the preferred embodiment, a polymer fiber 11 is wound in a single layer around the neck in contact with the mating surface (see Fig. 2). The implant 6 is heated using either a convection oven or an induction heater to a temperature around 800 degrees Fahrenheit. At this temperature, the layer of thermoplastic polymer fiber 11 melts into a coating 13, preferably around 10 thousands of an inch thick (see Fig. 3). The implant is allowed to cool which solidifies the thermoplastic. In the molten stage, the thermoplastic polymer is very sticky and adheres readily to the mating surface of the neck. When solid, the thermoplastic polymer is not tacky or sticky. In the preferred embodiment, the thermoplastic polymer of choice is from the polyaryletherketone family (PAEK). Specifically, a PAEK sold by BASF under the trade name Ultrapek@ A3000. The PAEK polymers have been shown through experimentation to exhibit strong adhesive bonds with molybdenum alloy (Co-Cr-Mo) and titanium-vanadium-aluminum alloy (Ti-6A1-4V) commonly used for implant materials.

To assemble the femoral head, the user places the head 12 on the neck 8 as illustrated in Fig. 4. At this point, the polymer coating 13 prevents the head 12 from fully seating on the neck 8. The assembly is then placed in a convection oven or induction heater and heated to a temperature of around 800 degrees Fahrenheit. When reheated, the thermoplastic polymer layer 13 remelts and exhibits its adhesive characteristic. With the thermoplastic polymer 13 melted, the user impacts the head 12 onto the neck 8 to fully seat the head as illustrated in Fig. 5. Once fully seated, the assembly is allowed to cool. When cooled, the thermoplastic polymer coating 13 creates a tight bond between the mating surfaces 10 and 16 of the head and neck. Experiments have shown the bond between these components to exhibit a strength of 10 ksi, essentially eliminating micro motion between the bonded parts. Further, since semi crystalline polymers such as the PAEK's are very moisture resistant, the coating of the mating surface of the neck and the eventual bond formed between the head and neck creates a moisture seal between the two components at their interface. Therefore, the corrosion which can occur at this junction is prevented. Finally, the coating of thermoplastic polymer forms a uniform contact surface between the mating surfaces of the parts to allow for stress transfer across the entire tapered surface of the neck. This acts to reduce the overall stress per unit area.

The steps of placing the head on the stem and heating the assembly as well as impacting the head on the stem can be carried out in the sterile environment of the operating room to permit the surgeon to choose from a variety of head sizes intraoperatively.

This method also has application to the connection of a thimble or an interpositional coupler or a neck extension between the femoral head and the neck of the implant. As illustrated in Fig. 6, the interpositional coupler 20, made from a similar material as the head 12 and a dissimilar material as the neck 8, would be bonded using the method of the invention directly to the neck 8 using a layer of thermoplastic polymer 13. Bonding the dissimilar materials together in the manufacturing facility would then permit the surgeon to perform the surgery in a normal fashion. Since the two dissimilar materials of the neck and interpositional coupler are bonded together using the thermoplastic polymer; and the similar materials of the coupler and head are impacted together in a standard manner; the corrosion potential should be held to a minimum.

It should be also understood that the method would work equally as well if the thermoplastic polymer were originally placed in contact with the mating surface of the femoral head cavity. While, it is believed such an alteration would be more difficult during manufacturing, it should be considered within the realm of the application.

Finally, it should be understood that the invention is not to be limited to the precise forms disclosed but may be modified within the scope of the appended claims.

## Claims

1. A method of bonding a neck of a femoral hip stem implant to a component for preventing corrosion therebetween, said neck having a predetermined geometry and including a mating surface, said component including a cavity having a geometry adapted for accommodating said neck, said cavity including a mating surface, said method comprising the steps of:
a) placing a thin layer of thermoplastic polymer on one of the mating surfaces;
b) placing the component on said neck such that the mating surfaces are in closely adjacent with said thin layer of thermoplastic polymer positioned between said mating surfaces;
c) applying heat to said neck, component and thermoplastic polymer sufficient to cause said thermoplastic polymer to become molten and adhere to said mating surfaces;
d) cooling said neck, component and thermoplastic polymer, wherein said thermoplastic polymer bonds said component to said neck and creates a seal therebetween for the prevention of motion between said neck and said component or the egress of moisture to thereby prevent corrosion of said mating surfaces.

2. The method of Claim 1 wherein step a comprises the steps of:
a) winding a thin fiber of the thermoplastic polymer around one of said mating surfaces;
b) applying heat to said one of said mating surfaces and to said thin fiber sufficient to cause said thin fiber to melt thereby forming a generally uniform layer of thermoplastic polymer.

3. The method of Claim 1 or claim 2 further including the step of;
a) impacting said component onto said neck after thermoplastic polymer becomes molten to fully seat the component onto the neck.

4. The method of any preceding Claim wherein said thermoplastic polymer is from the polyaryletherketone family.

5. The method of any preceding claim wherein said component is a prosthetic femoral head.

6. The method of any preceding claim wherein said component is an interpositional coupler.

7. A method for preventing corrosion between mating surfaces of a prosthetic femoral head and a neck of a prosthetic hip stem implant, said method comprising the steps of;
a) coating a mating surface of the neck with a thin film of thermoplastic polymer;
b) placing the head on the neck with the layer of thermoplastic polymer positioned therebetween;
c) applying heat to the neck, head and layer of thermoplastic polymer to cause said thermoplastic layer to become molten and bond to the head and neck;
d) impacting the head toward the neck to fully seat the head on the neck; and
e) allowing the head, neck and thermoplastic layer to cool wherein the thermoplastic layer creates a secure bond between the head and neck.

8. The method of Claim 7 wherein step (a) comprises the steps of;
a) winding a thin fiber of the thermoplastic polymer around mating surface of the neck;
b) applying heat to neck and to said thin fiber sufficient to cause said thin fiber to melt thereby forming a generally uniform layer of thermoplastic polymer.
